# EUROPEAN PATENT SPECIFICATION

(11) **EP 3 514 231 B1**
(45) Date of publication and mention of the grant of the patent: **12.06.2024**
(21) Application number: 17850955.0
(22) Date of filing: 14.09.2017
(51) Int. Cl.: C12N 11/082, C12N 11/087, C12N 9/90, C13K 13/00

(54) **METHOD FOR PRODUCING IMMOBILIZED ALLULOSE EPIMERASE**
VERFAHREN ZUR HERSTELLUNG VON IMMOBILISIERTER ALLULOSE-EPIMERASE
PROCÉDÉ DE PRODUCTION D'ALLULOSE ÉPIMÉRASE IMMOBILISÉE

(30) Priority: 14.09.2016 JP 2016179954
(43) Date of publication of application: 24.07.2019
(73) Proprietor: Matsutani Chemical Industry Co., Ltd., Itami-shi, Hyogo 664-8508 (JP)
(72) Inventor: GULLAPALLI, Pushpa Kiran, Itami-shi Hyogo 664-8508 (JP); SHINTANI, Tomoya, Itami-shi Hyogo 664-8508 (JP); NISHIOKA, Junichiro, Itami-shi Hyogo 664-8508 (JP); SHIMADA, Kensaku, Itami-shi Hyogo 664-8508 (JP)
(74) Representative: Müller-Boré & Partner Patentanwälte PartG mbB
(86) International application number: PCT/JP2017/033178
(87) International publication number: WO 2018/052054

(56) References cited:
- WO-A1-2008/142860
- WO-A1-2014/049373
- WO-A1-2015/032761
- WO-A1-2016/152819
- JP-A- 2001 011 090
- ZHANG WENLI ET AL: "Characterization of a D-psicose 3-epimerase from Dorea sp CAG317 with an acidic pH optimum and a high specific activity", JOURNAL OF MOLECULAR CATALYSIS B ENZYMATIC, vol. 120, October 2015 (2015-10), pages 68-74, XP002797124,
- CAN LI ET AL: "D-psicose 3-epimerase secretory overexpression, immobilization, and d-psicose biotransformation, separation and crystallization : D-psicose 3-epimerase secretory overexpression and d-psicose production", JOURNAL OF CHEMICAL TECHNOLOGY AND BIOTECHNOLOGY, vol. 93, no. 2, 30 June 2017 (2017-06-30), pages 350-357, XP55661241, ISSN: 0268-2575, DOI: 10.1002/jctb.5360
- TAKESHITA, K. et al.: "Mass Production of D-Psicose from D-Fructose by a Continuous Bioreactor System Using Immobilized D-Tagatose 3-Epimerase", Journal of Bioscience and Bioengineering, vol. 90, no. 4, 2000, pages 453-455, XP003003993, DOI: doi:10.1016/S1389-1723(01)80018-9
- LIM, B-C. et al.: "A stable immobilized D-psicose 3-epimerase for the production of D-psicose in the presence of borate", Process Biochemistry, vol. 44, 2009, pages 822-828, XP026160655, DOI: doi:10.1016/j.procbio.2009.03.017
- YOSHIHARA,A. et al.: "Purification and characterization of D-allulose 3-epimerase derived from Arthrobacter globifomis M30, a GRAS microorganism", Journal of Bioscience and Bioengineering, vol. 123, no. 2, 4 October 2016 (2016-10-04), pages 170-176, XP029896821, DOI: 10.1016/j.jbiosc.2016.09.004

## Description

The present invention relates to a method for producing an immobilized allulose epimerase. More specifically, the present invention relates to a method for efficiently producing an immobilized allulose epimerase having high specific activity and excellent durability.

Allulose produced by acting D-ketohexose 3-epimerase (Patent Document 1) on fructose is one kind of rare sugar also called psicose, and utilities such as its zero energy value (Non-Patent Document 1), effect of suppressing postprandial blood glucose elevation (Non-Patent Document 2), anti-obesity effect (Non-Patent Document 3), and the like have been reported and attracted attention as a lifestyle-related disease prevention material.

As described above, allulose is attracting attention as a diet sweetener, and the necessity of developing a method capable of efficiently producing allulose in the food industry field is increasing.

Conventionally, as a method for producing allulose, for example, a method of acting D-ketohexose 3-epimerase (hereinafter also referred to as "allulose epimerase") derived from microorganism using fructose as a substrate to enzymatically produce allulose is known.

As the allulose epimerase, allulose epimerase derived from Arthrobacter globiformis or Agrobacterium tumefaciens, tagatose epimerase derived from Pseudomonas cichorii or Rhodobacter sphaeroides, and the like are known.

In addition, when allulose is industrially produced using allulose epimerase, an immobilized allulose epimerase is used to increase production efficiency. Conventionally, as a method for producing an immobilized allulose epimerase, methods of using sodium alginate (Patent Document 2), a styrene-based porous-type weakly basic ion exchange resin (Patent Document 3) or a phenol-based porous-type weakly basic ion exchange resin (Patent Document 4) as an immobilization carrier are known.

Furthermore, Patent Document 5 discloses an improved variant of a D-psicose 3-epimerase and its uses. Patent Document 6 discloses a method for producing a novel sweetener containing glucose, fructose, and psicose, which is produced from glucose liquid sugar using an isomerase and an epimerase; use of the novel sweetener as a food or drink material; and a novel sweetener capable of preventing obesity caused by the intake thereof. Non-Patent Document 4 discloses charaterization of a D-psicose 3-epimerase from Dorea sp. CAG317 with an acidic pH optimum and a high specific activity. Non-Patent Document 5 concerns a mass productin of D-psicose from Dfrctose by a continuous bioreactor system using immobilized D-tagatose 3-epimerase. Non-Patent Document 6 discloses a stable immobilized D-psicose 3-epimerase for the production of D-psicose in the presence of borate.

However, in the conventional production method of an immobilized allulose epimerase, there are drawbacks such as insufficient performance such as economical efficiency (production cost) and stability (maintenance of activity) required for commercial production, and inability to withstand continuous production. Therefore, development of a method for efficiently producing an immobilized allulose epimerase having high specific activity and excellent durability is strongly desired.

Patent Document 1: JP H06-125776 A
Patent Document 2: JP 2013-501519 A
Patent Document 3: JP 2014-140361 A
Patent Document 4: JP 2015-530105 A
Patent Document 5: WO 2015/032761 A1
Patent Document 6: WO 2008/142860 A1

Non-Patent Document 1: J. Nutri Sci. Vitaminol., 48, 77-80, 2002.
Non-Patent Document 2: J. Nutri Sci. Vitaminol., 54, 511-514, 2008.
Non-Patent Document 3: Int. J. Food Sci. Nutri., 65, 245-250, 2014.
Non-Patent Document 4: Journal of Molecular Catalysis B: Enzymatic, vol. 120, 68-74, 2015.
Non-Patent Document 5: Journal of Bioscience and Bioengineering, vol. 90, 453-455, 2000.
Non-Patent Document 6: Process Biochemistry, vol. 44, 822-828, 2009.

An object of the present invention is to provide a method for efficiently producing an immobilized allulose epimerase having high specific activity and excellent durability.

For the purpose of solving the above problems, the present inventors have conducted intensive studies on a method capable of efficiently producing an immobilized allulose epimerase having high specific activity and excellent durability. As a result, it has been found that an immobilized allulose epimerase having high specific activity and excellent durability is efficiently obtained by bringing an enzyme solution containing allulose epimerase having a specific activity equal to or higher than a specific level into contact with a styrene-based porous-type weakly basic anion exchange resin or a styrene-based gel-type weakly basic anion exchange resin such that the total loaded protein amount is 1.3 to 15 mg/ml-R. The present invention has been accomplished by further studies based on such knowledge.

That is, the present invention is defined in the appended set of claims.

According to the present invention, it is possible to obtain an immobilized allulose epimerase having high allulose epimerase activity and excellent durability, as compared with conventional immobilized allulose epimerase. It is also possible to produce allulose by a simple method of passing a fructose solution through a column packed with the immobilized allulose epimerase obtained by the method of the present invention. Furthermore, by using a column combining the immobilized allulose epimerase of the present invention and existing immobilized glucose isomerase, it is also possible to efficiently produce allulose containing isomerized sugar from glucose.

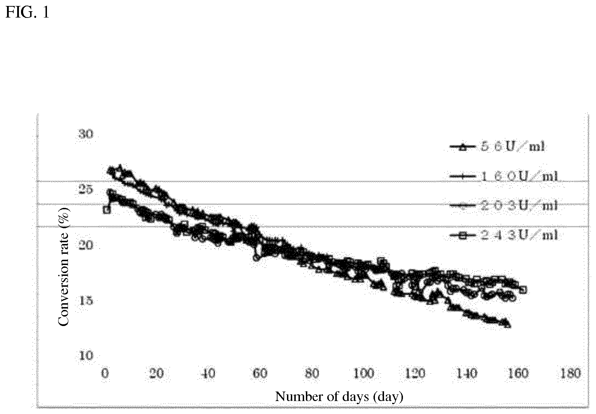

Fig. 1 is a result of evaluating the half-life of an immobilized allulose epimerase by carrying out sequential enzymatic reactions of fructose to allulose using the immobilized allulose epimerase in Example 3.

The present invention is a method for producing an immobilized allulose epimerase including a step of bringing an enzyme solution containing allulose epimerase having a specific activity of 50 U/mg or more into contact with a styrene-based porous-type weakly basic anion exchange resin or a styrene-based gel-type weakly basic anion exchange resin such that the total loaded protein amount is 2 to 15 mg/ml-R, wherein an ion exchange group of the styrene-based porous-type weakly basic anion exchange resin or the styrene-based gel-type weakly basic anion exchange is a tertiary amine, wherein the number of allulose epimerase units of the enzyme solution to be brought into contact with the ion exchange resin is 270 U or more per 1 ml of the ion exchange resin, wherein the specific activity of the immobilized allulose epimerase is 200 to 500 U/ml-R. Hereinafter, the production method of the present invention will be described in detail.

### [Definition]

In the present invention, the "enzyme activity (U) of allulose epimerase" is defined as 1 unit (U) as the enzymatic power for reacting allulose as a substrate at a reaction temperature of 50°C to produce 1 µmol of fructose per minute. Specifically, 2500 µl of a 0.2 M allulose solution dissolved in a 50 mM phosphate buffer (pH 8.0) containing 2 mM magnesium sulfate, 2167 µl of a 50 mM phosphate buffer (pH 8.0) containing 2 mM magnesium sulfate and 333 µl of allulose epimerase are placed in a screw capped test tube, and the test tube is immersed in a warm water bath and reacted at 50°C for 15 minutes. The pH of the reaction solution is adjusted to 2.5 to 3.0 by adding a 5% by mass aqueous hydrochloric acid solution to deactivate the enzyme, desalted with an ion exchange resin, filtered by filtration, and then analyzed by HPLC. The enzyme activity is calculated using a peak area ratio of the produced fructose.

In the present invention, the "specific activity (U/mg) of allulose epimerase" is the enzyme activity (U) of allulose epimerase of 1 mg of protein. Specifically, it can be determined by (1) preparing an enzyme solution in which allulose epimerase is dissolved, (2) measuring the protein concentration (mg/ml) of the enzyme solution by Bradford method, (3) measuring the enzyme activity (U/ml) of allulose epimerase of the enzyme solution by the above-mentioned method, and (4) dividing the enzyme activity (U/ml) of the obtained allulose epimerase by the protein concentration (mg/ml) to calculate a specific activity (U/mg) of allulose epimerase.

In the present invention, the "specific activity (U/ml-R) of the immobilized allulose epimerase" is an activity per 1 ml of an immobilized resin and is a value measured based on the following measurement method. Specifically, 2500 µl of a 0.2 M allulose solution dissolved in a 50 mM phosphate buffer (pH 8.0), 2480 µl of the 50 mM phosphate buffer (pH 8.0) and 20 mg of an immobilized allulose epimerase in a swollen state where unnecessary moisture is removed are placed in a 10 ml screw capped test tube, and the test tube was tilted horizontally and immersed in a warm water bath and reacted by shaking at 50°C for 15 minutes. Thereafter, the pH of the reaction solution is adjusted to 3.0 to 2.5 by adding a 5% by mass aqueous hydrochloric acid solution to deactivate the enzyme, desalted with an ion exchange resin, filtered by filtration, and then analyzed by HPLC. The specific activity (U/ml-R) of the immobilized allulose epimerase is obtained from the peak area ratio of the produced fructose.

In the present invention, the "total loaded protein amount (mg/ml-R)" refers to a value obtained by dividing the mass (mg) of the protein contained in the total amount of the enzyme solution to be brought into contact by the capacity (1 ml-R) of the ion exchange resin brought into contact with the enzyme solution in a swollen state.

In the present invention, the "space velocity (SV)" refers to a unit of the speed at which a solution is passed through a column, and is calculated by "space velocity (SV) = amount of passed liquid (ml)/column volume (ml)/hour (h)".

### [Allulose Epimerase]

Allulose epimerase is an enzyme capable of catalyzing an interconversion between fructose and allulose. The allulose epimerase used in the present invention is not particularly limited by its origin and may be derived from any organisms such as microorganisms, animals, and plants. For example, it is known that allulose epimerase is produced by microorganisms such as Arthrobacter globiformis M30 strain (deposit number: NITE BP-1111), Pseudomonas cichorii, Agrobacterium tumefaciens, Clostrideum sp., Clostridium scindens, Clostrideum bolteae, Clostridium cellulolyticum, and Ruminococcus sp.

In addition, the allulose epimerase to be immobilized in the present invention may be one produced using the above organism, or it may be a recombinant allulose epimerase produced by a genetic engineering technique. Furthermore, the allulose epimerase used in the present invention may be a mutant obtained by mutating an allulose epimerase derived from the organism.

In addition, the allulose epimerase to be immobilized in the present invention may be either a purified product or a roughly purified product.

### [Enzyme Solution to Be Immobilized]

In the present invention, an enzyme solution containing allulose epimerase having a specific activity of 50 U/mg or more is to be immobilized to an immobilization carrier described below. As described above, by immobilizing allulose epimerase with a specific total loaded protein amount using an enzyme solution having a specific specific activity and the immobilization carrier described below, it is possible to efficiently produce an immobilized allulose epimerase having high specific activity and excellent durability.

The specific activity of allulose epimerase of the enzyme solution to be immobilized on the immobilization carrier is not particularly limited as far as it is 50 U/mg or more. However, the specific activity is preferably 50 U/mg to 200 U/mg, and further preferably 50 to 160 U/mg, from the viewpoint of more efficiently producing an immobilized allulose epimerase having high specific activity and excellent durability.

The solvent of the enzyme solution to be immobilized on the immobilization carrier is not particularly limited and may be any of water, buffer solution and the like.

### [Immobilization Carrier]

The immobilization carrier of allulose epimerase used in the present invention is a styrene-based porous-type weakly basic anion exchange resin or a styrene-based gel-type weakly basic anion exchange resin. By using such a specific anion exchange resin, it is possible to efficiently produce an immobilized allulose epimerase having high specific activity and excellent durability.

The styrene-based porous-type weakly basic anion exchange resin refers to a porous weakly basic anion exchange resin having holes (macropores) physically opened consisting of a gel-like resin substrate of a copolymer of polystyrene divinylbenzene.

The form of the styrene-based porous-type weakly basic anion exchange resin is not particularly limited, and it may be, for example, any of powder, spherical, fibrous, filmy, and the like.

The ion exchange group in the styrene-based porous-type weakly basic anion exchange resin is a tertiary amine, which is an anion exchangeable group showing weak basicity. These ion exchange groups may be contained in a styrene-based porous-type weakly basic anion exchange resin by one type singly or may be contained in a styrene-based porous-type weakly basic anion exchange resin in combination of two or more types. Among these ion exchange groups, a tertiary amine is preferable, and a group - N(CH₃)₂ is further preferable, from the viewpoint of more efficiently producing an immobilized allulose epimerase having high specific activity and excellent durability.

As the styrene-based porous-type weakly basic anion exchange resin, for example, Urbanlite EPA95, Amberlite IRA904 (manufactured by Organo Corporation); Duolite A378D (manufactured by Sumika Chemtex Co., Ltd.); Purolite A111S, Purolite A103S (manufactured by Purolite); DIAION WA20, DIAION WA30 (manufactured by Mitsubishi Rayon Aqua Solutions Co., Ltd.) and the like are commercially available, and these commercially available products can also be used in the present invention.

The styrene-based gel-type weakly basic anion exchange resin is a weakly basic anion exchange resin consisting of a gel-like resin substrate of a copolymer of polystyrene divinylbenzene.

The form of the styrene-based gel-type weakly basic anion exchange resin is not particularly limited, and it may be, for example, any of powder, spherical, fibrous, filmy, and the like.

The ion exchange group in the styrene-based gel-type weakly basic anion exchange resin is not particularly limited as far as it is an anion exchangeable group showing weak basicity. Examples thereof include amines such as tertiary amine, quaternary amine, and polyamines. These ion exchange groups may be contained in a styrene-based gel-type weakly basic anion exchange resin by one type singly or may be contained in a styrene-based gel-type weakly basic anion exchange resin in combination of two or more types. Among these ion exchange groups, a tertiary amine is preferable from the viewpoint of more efficiently producing an immobilized allulose epimerase having high specific activity and excellent durability.

As the styrene-based gel-type weakly basic anion exchange resins, for example, DIAION HPA25L (manufactured by Mitsubishi Rayon Aqua Solutions Co., Ltd.), Amberlite IRA411S (manufactured by Organo Corporation) and the like are commercially available, and these commercially available products can also be used in the present invention.

In the styrene-based porous-type weakly basic anion exchange resin and the styrene-based gel-type weakly basic anion exchange resin, the total exchange capacity (the maximum ion exchange amount of the ion exchange resin) is not particularly limited, but is usually 1 eq/l-R or more, preferably 1 to 2 eq/l-R, and further preferably 1.2 to 1.5 eq/l-R, from the viewpoint of more efficiently producing an immobilized allulose epimerase having high specific activity and excellent durability.

In the present invention, either one of a styrene-based porous-type weakly basic anion exchange resin or a styrene-based gel-type weakly basic anion exchange resin may be used, or both of them may be used in combination.

Among these anion exchange resins, a styrene-based porous-type weakly basic anion exchange resin is preferable, and a styrene-based porous-type weakly basic anion exchange resin having a tertiary amine as an ion exchange group is further preferable, from the viewpoint of more efficiently producing an immobilized allulose epimerase having high specific activity and excellent durability.

### [Immobilization of Allulose Epimerase]

By bringing the enzyme solution into contact with the immobilization carrier such that the total loaded protein amount is 2 to 15 mg/ml-R, an immobilized allulose epimerase having high specific activity of allulose epimerase and excellent durability is obtained.

Prior to bringing the enzyme solution into contact with the immobilization carrier, it is desirable to wash the immobilization carrier using a washing solution such as a buffer solution.

The amount of the enzyme solution to be brought into contact with the immobilization carrier is set such that the total loaded protein amount is 2 to 15 mg/ml-R. However, the total loaded protein amount of the enzyme solution is preferably 3 to 10 mg/ml-R, and further preferably 5 to 10 mg/ml-R, from the viewpoint of efficiently producing an immobilized allulose epimerase having high specific activity.

In the step of bringing the immobilization carrier into contact with the enzyme solution, the ratio of the amount of allulose epimerase contained in the enzyme solution and the immobilization carrier may be appropriately set within a range that can satisfy the range of the total loaded protein amount. Examples include ratios such that the total amount of allulose epimerase contained in the enzyme solution to be brought into contact per a volume of 1 ml of the immobilization carrier in the swollen state is 200 to 2000 U, preferably 500 to 1000 U, and further preferably 600 to 900 U, from the viewpoint of more efficiently producing an immobilized allulose epimerase having high specific activity.

The method of bringing the enzyme solution into contact with the immobilization carrier is not particularly limited, and a method adopted in the production of ordinary immobilized enzyme may be used. Specific examples include a method of passing the enzyme solution through a column packed with the immobilization carrier, a method of adding the immobilization carrier to a container containing the enzyme solution, and the like.

In the case of the method of passing the enzyme solution through a column packed with the immobilization carrier, the space velocity of the enzyme solution to the column packed with the immobilization carrier is not particularly limited, but is, for example, from 0.1 to 1.0 hr⁻¹, preferably from 0.2 to 0.8 hr⁻¹, and further preferably from 0.4 to 0.6 hr⁻¹. Also, in the case of producing an immobilized allulose epimerase by the method, it is desirable that the enzyme solution discharged by passing through the column is again passed through the column, whereby the enzyme solution is circulated and the enzyme solution is repeatedly passed.

In addition, in the case of the method of passing the enzyme solution through a column packed with the immobilization carrier, the time for passing the enzyme solution is not particularly limited, but is, for example, 5 to 20 hours, preferably 10 to 18 hours, and further preferably 12 to 16 hours.

Moreover, in the case of the method of adding the immobilization carrier to a container containing the enzyme solution, the immobilization carrier is added to a container containing the enzyme solution, and the mixture is stirred as necessary, and should be incubated until allulose epimerase is immobilized on the immobilization carrier.

Further, in the case of the method of passing the enzyme solution through a column packed with the immobilization carrier, the time for adding the immobilization carrier to the container containing the enzyme solution and incubating is not particularly limited, but is, for example, 10 to 40 hours, preferably 15 to 35 hours, and further preferably 20 to 30 hours.

The immobilized allulose epimerase thus obtained may be washed using a washing solution such as a buffer solution, as necessary. In addition, the obtained immobilized allulose epimerase may be crosslinked with glutaraldehyde, polyethyleneimine or the like, as necessary, to strengthen immobilization of allulose epimerase.

### [Characteristic and Application of Immobilized Allulose Epimerase]

The immobilized allulose epimerase thus obtained has high specific activity of allulose epimerase and can have excellent durability.

The specific activity of the immobilized allulose epimerase obtained by the production method of the present invention is 200 to 500 U/ml-R, and preferably 250 to 500 U/ml-R.

Also, as the durability of the immobilized allulose epimerase obtained by the production method of the present invention, the half-life of the specific activity of the immobilized allulose epimerase under the following test conditions is 150 days or more, preferably 160 to 250 days, and further preferably 200 to 240 days.

### (Durability Test Conditions)

A jacketed glass column (inner diameter: 20 mm, length: 400 mm) is packed with an immobilized allulose epimerase whose activity is equivalent to 4500 U. Separately, 2 mM magnesium sulfate is added and sodium carbonate is further added to prepare a 35% by mass fructose solution adjusted to a pH of 7.8 to 8.0. The fructose solution is continuously brought into contact with the glass column packed with the immobilized allulose epimerase at a jacket temperature of 55°C in an upward flow such that the fructose content is 0.004 g/hr/U. For example, in the case of an immobilized allulose epimerase having a specific activity of 450 U/ml-R, 10 ml of an immobilized allulose epimerase is packed in a jacketed glass column (inner diameter 20 mm, length 400 mm), and the fructose solution is passed through a space velocity of 5 hr⁻¹. An outflow liquid flowing out from the column was sampled once every 24 hours, each of the collected solutions was desalted, filtered and then analyzed by HPLC to determine an area of allulose occupied in a peak area of fructose and allulose, and it was defined as a conversion rate. An approximate straight line is obtained by plotting the conversion rate over time, and the number of days which is half of the conversion efficiency one day after the start of the test is determined from the approximate straight line and defined as the half-life.

As described above, the immobilized allulose epimerase obtained by the production method of the present invention has high specific activity of allulose epimerase and has excellent durability, so that it is suitable to industrially and continuously produce allulose from fructose or glucose. Also, the immobilized allulose epimerase obtained by the production method of the present invention may be used alone for the production of allulose or may be used in combination with other immobilized enzyme (for example, immobilized glucose isomerase or the like) for the production of allulose.

### EXAMPLES

Hereinafter, the present invention will be described in more detail by showing examples, but the present invention is not limited thereto.

### Example 1 (Screening of Immobilization Carrier)

### 1. Preparation of allulose epimerase used for immobilization

Allulose epimerase used for immobilization was prepared through the steps shown in the following (1) and (2).

### (1) Cell culture and cell collection

Arthrobacter globiformis M30 strain was inoculated into 4 L of minimal salt medium (MSM medium) containing 0.5% by mass allulose and incubated at 30°C for 24 hours using a jar fermenter, with a stirring speed of 400 rpm, and an airflow rate per minute of 0.10 L/medium L. From this culture solution, 100 g (wet weight) of cells was recovered by centrifugation and washed with a 50 mM phosphate buffer (pH 8.0).

### (2) Extraction step of crude enzyme

100 g (wet weight) of the obtained cells was suspended in 1000 ml of a 50 mM phosphate buffer (pH 8.0), 10 g of egg white lysozyme (food additive, manufactured by Kewpie Corporation) and 5 g of sodium chloride were added thereto, and the mixture was heated at 37°C for 120 minutes to perform extraction reaction of an enzyme. Thereafter, heating was further performed at 55°C for 15 minutes, and the supernatant obtained by centrifugation (12000 rpm, 30 minutes) was used as a crude enzyme solution. The specific activity of allulose epimerase per 1 mg of the total protein contained in the crude enzyme solution was 4.9 U/mg.

### 2. Primary screening of immobilization carriers

As an immobilization carrier of allulose epimerase, ion exchange resins (commercially available products) shown in Table 1 were evaluated.

**[Table 1]**

| Ion exchange resins and details thereof | | | | | | |
|---|---|---|---|---|---|---|
| No. | Structure of resin matrix | Ion exchange group | Type* | Total exchange capacity (eq/1) | Ion type | Name (manufacturer**) |
| 1 | Styrene-based porous-type | Tertiary amine | WA | ≥ 1.25 | Free | Amberlite FPA95 (O) |
| 2 | Styrene-based porous-type | Tertiary amine (90%) Quaternary ammonium (10%) | WA | 1.3 | Free | Duolite A378D (S) |
| 3 | Styrene-based porous-type | Tertiary amine | WA | ≥ 1.7 | Free | Purolite A111S (P) |
| 4 | Styrene-based porous-type | Tertiary amine | WA | ≥ 1.5 | Free | Purolite A103S (P) |
| 5 | Styrene-based porous-type | Quaternary amine | SA | ≥ 0.65 | Cl | Amberlite IRA904 (O) |
| 6 | Styrene-based gel-type | Tertiary amine | WA | ≥ 0.5 | Free | DIAION HPA25L (M) |
| 7 | Styrene-based porous-type | Polyamine | WA | ≥ 2.5 | Free | DIAION WA20 (M) |
| 8 | Styrene-based gel-type | Quaternary amine | SA | ≥ 0.9 | Cl | Amberlite IRA411S (O) |
| 9 | Acrylic-based gel-type | Quaternary amine | SA | ≥ 0.8 | Cl | Amberlite IRA958 (O) |
| 10 | Acrylic-based gel-type | Polyamine | WA | ≥ 1.2 | Free | DIAION WA10 (M) |
| 11 | Acrylic-based porous-type | Unknown | WA | Unknown | Unknown | KA895 (S) |

| | | | | | | |
|---|---|---|---|---|---|---|
| * WA: weakly basic anion exchanger, SA: strongly basic anion exchanger ** (O): Organo Corporation, (S): Sumika Chemtex Co., Ltd., (P): Purolite, (M): Mitsubishi Rayon Aqua Solutions Co., Ltd. | | | | | | |

355 mg of the ion exchange resin of Table 1 washed with a 50 mM phosphate buffer (pH 8.0) containing 2 mM magnesium sulfate and 10 ml of a 50 mM phosphate buffer (pH 8.0) containing 30 U of the prepared allulose epimerase were mixed and slowly stirred in a chamber set at 20°C for 24 hours using a twist mixer. Subsequently, the supernatant was removed using a pipette, and the resulting mixture was washed 5 times with 10 ml of a 50 mM phosphate buffer (pH 8.0) to obtain an immobilized allulose epimerase.

The method of measuring the enzyme activity of immobilized allulose epimerase in the primary screening is as follows. That is, 500 µl of a 0.2 M allulose solution dissolved in a 50 mM phosphate buffer (pH 8.0), 480 µl of a 50 mM phosphate buffer (pH 8.0) and 20 mg of immobilized allulose epimerase from which unnecessary moisture was removed with a paper cloth (Kimwipe) was placed in a microtube, and immersed in a warm water bath and reacted at 50°C for 10 minutes. Thereafter, the enzyme was deactivated by putting it in a boiling bath at 95°C for 5 minutes, desalted with an ion exchange resin, filtered by filtration, and then analyzed by HPLC (analytical column: MCIGEL CK08EC, manufactured by Mitsubishi Chemical Corporation). The specific activity (U/ml-R) of the immobilized allulose epimerase was determined from the peak area ratio of fructose to the total peak area of fructose and allulose.

The obtained results are shown in Table 2. As a result, there was a tendency that the specific activity of allulose epimerase immobilized with the styrene-based porous-type weakly basic ion exchange resin and the styrene-based gel-type weakly basic type ion exchange resin was high, in particular, the specific activity of allulose epimerase immobilized with the ion exchange resin having a tertiary amine in the styrene-based porous-type, and the ion exchange resin having a tertiary amine in the styrene-based gel-type was high. However, although not shown in the data, it was found that, even in the case of a styrene-based porous-type tertiary amine in another experiment in which an immobilized allulose epimerase was acted on fructose to continuously produce allulose, a strongly basic anion exchange resin decomposes the produced allulose and lowers the purity, thus it was found unsuitable as an immobilization carrier.

**[Table 2]**

| Specific activity of immobilized allulose epimerase | | | | | |
|---|---|---|---|---|---|
| No. | Type of resin matrix | Ion exchange group | Type | Ion type | Specific activity (U/ml-R) |
| 1 | Styrene-based porous-type | Tertiary amine | WA | Free | 14 |
| 2 | Styrene-based porous-type | Tertiary amine (90%) Quaternary ammonium (10%) | WA | Free | 15 |
| 3 | Styrene-based porous-type | Tertiary amine | WA | Free | 16 |
| 4 | Styrene-based porous-type | Tertiary amine | WA | Free | 15 |
| 5 | Styrene-based porous-type | Quaternary amine | SA | Cl | 15 |
| 6 | Styrene-based gel-type | Tertiary amine | WA | Free | 19 |
| 7 | Styrene-based porous-type | Polyamine | WA | Free | 6 |
| 8 | Styrene-based gel-type | Quaternary amine | SA | Cl | 1 |
| 9 | Acrylic-based gel-type | Quaternary amine | SA | Cl | 1 |
| 10 | Acrylic-based gel-type | Polyamine | WA | Free | 1 |
| 11 | Acrylic-based porous-type | Unknown | WA | Unknown | 4 |

### 3. Secondary screening of immobilization carriers

Next, for the ion exchange resins Nos. 1 and 3 which had high specific activity in the primary screening and were evaluated as efficient, the number of units of allulose epimerase used for immobilization was increased and the adsorption immobilization capacity was evaluated.

Allulose epimerase that was produced using an E. coli expression system was used. That is, an allulose epimerase gene derived from Arthrobacter globiformis M30 strain was incorporated into a pQE vector (QIAGEN), which was incorporated into E. coli M15 (QIAGEN) for expression, and 16 g of the obtained cells was suspended in 80 ml of a 50 mM phosphate buffer (pH 8.0) containing 2 mM magnesium sulfate and extracted and purified by ultrasonication and centrifugation. The specific activity of the obtained allulose epimerase was 93.8 U/mg.

1 ml of the ion exchange resin No. 1 or 3 shown in Table 1 washed with a 50 mM phosphate buffer (pH 8.0) containing 2 mM magnesium sulfate and 10 ml of a 50 mM phosphate buffer (pH 8.0) containing 450 U or 1000 U of allulose epimerase were mixed and slowly stirred in a chamber set at 20°C for 24 hours using a twist mixer. Thereafter, the supernatant was removed using a pipette, and the resulting mixture was washed 5 times with 10 ml of a 50 mM phosphate buffer (pH 8.0) to obtain an immobilized allulose epimerase. The method of measuring the enzyme activity of immobilized allulose epimerase in the secondary screening is as follows. That is, 2500 µl of a 0.2 M allulose solution dissolved in a 50 mM phosphate buffer (pH 8.0), 2480 µl of the 50 mM phosphate buffer (pH 8.0) and 20 mg of an immobilized allulose epimerase from which unnecessary moisture was removed with a paper cloth (Kimwipe) was placed in a 10 ml screw capped test tube, and the test tube was placed horizontally in a warm water bath and reacted by shaking at 50°C for 15 minutes. The pH of the reaction solution is adjusted to 3.0 to 2.5 by adding a 5% by mass aqueous hydrochloric acid solution to deactivate the enzyme, desalted with an ion exchange resin, filtered by filtration, and then analyzed by HPLC (analytical column: MCIGEL CK08EC, manufactured by Mitsubishi Chemical Corporation). The specific activity (U/ml-R) of the immobilized allulose epimerase was determined from the peak area ratio of fructose to the total peak area of fructose and allulose.

The obtained results are shown in Table 3. As a result, it was unexpectedly found that when the number of loaded enzyme units was increased, the specific activity of the immobilized allulose epimerase increased. Moreover, it was also revealed that the ion exchange resin giving an immobilized allulose epimerase having the highest specific activity was an ion exchange resin (Amberlite FPA95 manufactured by Organo Corporation) shown in No. 1 of Table 1.

**[Table 3]**

| No. | Structure of resin matrix | Ion exchange group | Specific activity (U/ml-R) | |
|---|---|---|---|---|
| | | | 450U* | 1000U* |
| 1 | Styrene-based porous-type | Tertiary amine | 170 | 235 |
| 3 | Styrene-based porous-type | Tertiary amine | 169 | 211 |

| | | | | |
|---|---|---|---|---|
| * The number of allulose epimerase units loaded per 1 ml of ion exchange resin | | | | |

Furthermore, allulose epimerase was immobilized using the ion exchange resin (Amberlite FPA95 manufactured by Organo Corporation) shown in No. 1 of Table 1, by changing the loaded enzyme unit of allulose epimerase to be immobilized. Specifically, 1 ml of the ion exchange resin No. 1 or 3 shown in Table 1 washed with a 50 mM phosphate buffer (pH 8.0) containing 2 mM magnesium sulfate and 10 ml of a 50 mM phosphate buffer (pH 8.0) containing 540 U, 630 U, 900 U, 1350 U or 1800 U of allulose epimerase having a specific activity of 93.8 U/mg were mixed and slowly stirred in a chamber set at 20°C for 24 hours using a twist mixer. Thereafter, the supernatant was removed using a pipette, and the resulting mixture was washed 5 times with 10 ml of a 50 mM phosphate buffer (pH 8.0) to prepare an immobilized allulose epimerase, and the specific activity of the immobilized allulose epimerase (U/ml-R) and the activity (U/ml) of the enzyme solution remaining after immobilization were measured. Specifically, 2500 µl of a 0.2 M allulose solution dissolved in a 50 mM phosphate buffer (pH 8.0), 2480 µl of the 50 mM phosphate buffer (pH 8.0) and 20 mg of an immobilized allulose epimerase from which unnecessary moisture was removed with a paper cloth (Kimwipe) was placed in a 10 ml screw capped test tube, and the test tube was placed horizontally in a warm water bath and reacted by shaking at 50°C for 15 minutes. The pH of the reaction solution is adjusted to 3.0 to 2.5 by adding a 5% by mass aqueous hydrochloric acid solution to deactivate the enzyme, desalted with an ion exchange resin, filtered by filtration, and then analyzed by HPLC (analytical column: MCIGEL CK08EC, manufactured by Mitsubishi Chemical Corporation). The specific activity (U/ml-R) of the immobilized allulose epimerase was determined from the peak area ratio of fructose to the total peak area of fructose and allulose. In addition, 2500 µl of a 0.2 M allulose solution dissolved in a 50 mM phosphate buffer (pH 8.0) containing 2 mM magnesium sulfate, 2167 µl of a 50 mM phosphate buffer (pH 8.0) containing 2 mM magnesium sulfate and 333 µl of a remaining enzyme solution are placed in a screw capped test tube, and the test tube is immersed in a warm water bath and reacted at 50°C for 15 minutes. The pH of the reaction solution is adjusted to 2.5 to 3.0 by adding a 5% by mass aqueous hydrochloric acid solution to deactivate the enzyme, desalted with an ion exchange resin, filtered by filtration, and then analyzed by HPLC. The residual enzyme activity (U/ml) was determined using the peak area ratio of the produced fructose.

The obtained results are shown in Table 4. As a result, it was found that an immobilized allulose epimerase having high specific activity can be efficiently produced when the total loaded protein amount is in the range of 1.3 to 15 mg/ml-R.

**[Table 4]**

| Number of loaded enzyme units (U)* | Loaded protein amount (mg/ml-R) | Specific activity of immobilized allulose epimerase (U/ml-R) | Residual enzyme activity (U/ml) |
|---|---|---|---|
| 120 | 1.3 | 89 | 0.37 |
| 270 | 2.9 | 153 | 0.33 |
| 540 | 5.8 | 227 | 0.41 |
| 630 | 6.7 | 243 | 0.34 |
| 900 | 9.6 | 249 | 0.27 |
| 1350 | 14.4 | 243 | 3.1 |
| 1800 | 19.2 | 248 | 25.6 |

| | | | |
|---|---|---|---|
| * The number of allulose epimerase units loaded per 1 ml of ion exchange resin | | | |

### 4. Immobilization of other microorganism-derived allulose epimerase

For the ion exchange resins of Nos. 1 and 3 in Table 1, the adsorption immobilization capacity of other microorganism-derived allulose epimerase was similarly evaluated.

For other microorganism-derived allulose epimerase, Clostridium Cellulolyticum H10 strain-derived allulose epimerase was used. That is, the allulose epimerase gene synthesized by Life Technologies Corporation was incorporated into a pQE vector (QIAGEN), which was incorporated into E. coli M15 (QIAGEN) for expression, and 16 g of the obtained cells was suspended in 80 ml of a 50 mM phosphate buffer (pH 8.0) containing 2 mM magnesium sulfate and extracted and purified by ultrasonication and centrifugation. The specific activity of the obtained allulose epimerase was 156 U/mg.

1 ml of the ion exchange resins of Nos. 1 and 3 in Table 1 washed with a 50 mM phosphate buffer (pH 8.0) containing 2 mM magnesium sulfate and 10 ml of a 50 mM phosphate buffer (pH 8.0) containing 630 U of allulose epimerase (6.7 mg in terms of protein amount) were mixed and slowly stirred in a chamber set at 20°C for 24 hours using a twist mixer. Subsequently, the supernatant was removed using a pipette, and the resulting mixture was washed 5 times with 10 ml of a 50 mM phosphate buffer (pH 8.0) to obtain an immobilized allulose epimerase. The specific activity of the immobilized allulose epimerase was measured in the same manner as in the secondary screening.

The obtained results are shown in Table 5. From this result, as in the secondary screening, the ion exchange resin giving the immobilized allulose epimerase having the highest specific activity was the ion exchange resin (Amberlite FPA95 manufactured by Organo Corporation) shown in No. 1 of Table 1.

**[Table 5]**

| No. | Structure of resin matrix | Ion exchange group | Specific activity (U/ml-R) |
|---|---|---|---|
| 1 | Styrene-based porous-type | Tertiary amine | 506 |
| 3 | Styrene-based porous-type | Tertiary amine | 468 |

### Example 2 (Production of Immobilized Allulose Epimerase)

1. Production of immobilized low-activity allulose epimerase (Comparative Example) Immobilized low-activity allulose epimerase was prepared through the steps shown in the following (1) to (3).

### (1) Cell culture and cell collection step

Arthrobacter globiformis M30 strain was inoculated into 4 L of minimal salt medium (MSM medium) containing 0.5% by mass allulose and incubated at 30°C for 24 hours using ajar fermenter, with a stirring speed of 400 rpm, and an airflow rate per minute of 0.10 L/medium L. From this culture solution, 100 g (wet weight) of cells was recovered by centrifugation and washed with a 50 mM phosphate buffer (pH 8.0).

### (2) Extraction step of crude enzyme

100 g (wet weight) of the obtained cells was suspended in 1000 ml of a 50 mM phosphate buffer (pH 8.0), 10 g of egg white lysozyme (food additive, manufactured by Kewpie Corporation) and 5 g of sodium chloride were added thereto, and the mixture was heated at 37°C for 120 minutes to perform extraction reaction of an enzyme. Thereafter, heating was further performed at 55°C for 15 minutes, and the supernatant obtained by centrifugation (12000 rpm, 30 minutes) was used as a crude enzyme solution. The specific activity of allulose epimerase per 1 mg of the total protein contained in the crude enzyme solution was 4.9 U/mg.

### (3) Immobilization of allulose epimerase

50 ml of a wet state ion exchange resin (ion exchange resin shown in No. 4 of Table 1, manufactured by Purolite Co., Ltd., trade name: Purolite A103S) was packed in a column and washed with a 50 mM phosphate buffer (pH 8.0) containing 2 mM magnesium sulfate, then 500 ml of a 2 mM magnesium sulfate-containing 50 mM phosphate buffer (pH 8.0) containing 4500 U (918 mg in terms of protein amount) of the allulose epimerase prepared above was passed through the ion exchange resin at 4°C for 16 hours to adsorb the enzyme while circulating, followed by washing with a 2 mM magnesium sulfate-containing 50 mM phosphate buffer (pH 8.0), to obtain an immobilized allulose epimerase. The amount of protein loaded at this time was 18.3 mg/ml-R. The specific activity of the obtained immobilized allulose epimerase was 56 U/ml-R.

### 2. Production of immobilized high-activity allulose epimerase

Allulose epimerase that was produced using an E. coli expression system was used. That is, an allulose epimerase gene derived from Arthrobacter globiformis M30 strain was incorporated into a pQE vector (QIAGEN), which was incorporated into E. coli M15 strain (QIAGEN) for expression, and 16 g of the obtained cells was suspended in 160 ml of a 50 mM phosphate buffer (pH 8.0) containing 2 mM magnesium sulfate (pH 8.0) and extracted and purified by ultrasonication and centrifugation to obtain an allulose epimerase solution. The specific activity of allulose epimerase per 1 mg of the total protein contained in the obtained allulose epimerase solution was 51.9 U/mg.

50 ml of a wet state ion exchange resin (ion exchange resin indicated by No. 1 in Table 1, manufactured by Organo Corporation, trade name: Amberlite FPA95) was packed in a column and washed with a 50 mM phosphate buffer (pH 8.0) containing 2 mM magnesium sulfate, then 500 ml of a 2 mM magnesium sulfate-containing 50 mM phosphate buffer (pH 8.0) containing 13500 U (260 mg in terms of protein amount), 22500 U (433 mg in terms of protein amount) or 31500 U (607 mg in terms of protein amount) of the allulose epimerase prepared above (pH 8.0) was loaded onto the ion exchange resin at 4°C for 16 hours to adsorb the enzyme while circulating, followed by washing with a 2 mM magnesium sulfate-containing 50 mM phosphate buffer (pH 8.0), to obtain an immobilized allulose epimerase. The specific activities of the obtained immobilized allulose epimerase were 160 U/ml-R, 203 U/ml-R, and 243 U/ml-R, respectively.

### Example 3 (Continuous Use of Immobilized Allulose Epimerase)

Sequential enzymatic reactions were carried out, using the immobilized low-activity allulose epimerase (56 U/ml-R) obtained in Example 2 and the immobilized high-activity allulose epimerase (160 U/ml-R, 203 U/ml-R, 243 U/ml-R), respectively, and the half-life of the enzymatic reaction was evaluated.

80 ml of immobilized low-activity allulose epimerase of 56 U/ml-R, 28 ml of immobilized high-activity allulose epimerase of 160 U/ml-R as a reference example, 22 ml of immobilized high-activity allulose epimerase of 203 U/ml-R, and 18.5 ml of immobilized high-activity allulose epimerase of 243 U/ml-R were packed in a jacketed glass column (inner diameter 20 mm, length 400 mm), respectively. Also, separately, magnesium sulfate was added to 2 mM, and sodium carbonate was further added to adjust the pH to 7.8 to 8.0 to prepare a 35% by mass fructose solution. The fructose solution was passed through the column continuously for up to a total of 4320 hours at a jacket temperature of 55°C in an upward flow. In order to bring the fructose of 0.007 g/h per the immobilized allulose epimerase amount (1 U) into contact, the space velocity was set to SV = 1 in the case of the immobilized low-activity allulose epimerase, the space velocity was set to SV = 3 in the case of the immobilized high-activity allulose epimerase of 160 U/ml-R as a reference example, the space velocity was set to SV = 3.6 in the case of the immobilized high-activity allulose epimerase of 203 U/ml-R, and the space velocity was set to SV = 4.3 in the case of the immobilized high-activity allulose epimerase of 243 U/ml-R, respectively, and the fructose solution was passed through. An outflow liquid was sampled once every 24 hours, a part of each collected solution was desalted with an ion exchange resin, filtered with a filter, and then subjected to HPLC (analytical column: MCIGEL CK08EC, manufactured by Mitsubishi Chemical Corporation) analysis to determine an area of allulose occupied in a peak area of fructose and allulose, and it was defined as a conversion rate. An approximate straight line was obtained by plotting the conversion rate over time, and the number of days which was half of the conversion efficiency one day after the start of the test was determined from the approximate straight line and calculated as the half-life.

The obtained results are shown in Fig. 1. As a result, unexpectedly, the half-life of the immobilized low-activity allulose epimerase of 56 U/ml-R was 144 days, whereas the half-life of the immobilized high-activity allulose epimerase of 160 U/ml-R as a reference example was 154 days, the half-life of the immobilized high-activity allulose epimerase of 203 U/ml-R was 207 days, and the half-life of the immobilized high-activity allulose epimerase of 243 U/ml-R was 237 days. That is, it was revealed that the immobilized allulose epimerase having high specific activity has a long half-life, excellent durability, and more suitable for continuous reaction.

## Claims

1. A method for producing an immobilized allulose epimerase comprising a step of bringing an enzyme solution containing allulose epimerase having a specific activity of 50 U/mg or more into contact with a styrene-based porous-type weakly basic anion exchange resin or a styrene-based gel-type weakly basic anion exchange resin such that the total loaded protein amount is 2 to 15 mg/ml-R,
wherein an ion exchange group of the styrene-based porous-type weakly basic anion exchange resin or the styrene-based gel-type weakly basic anion exchange is a tertiary amine,
wherein the number of allulose epimerase units of the enzyme solution to be brought into contact with the ion exchange resin is 270 U or more per 1 ml of the ion exchange resin,
wherein the specific activity of the immobilized allulose epimerase is 200 to 500 U/ml-R, and
wherein the specific activity of allulose epimerase is measured according to the method disclosed in the description.

2. The method according to claim 1, wherein an ion exchange group of the styrene-based porous-type weakly basic anion exchange resin is -N(CH₃)₂.

## Patentansprüche

1. Verfahren zur Herstellung einer immobilisierten Allulose-Epimerase, umfassend einen Schritt des Inkontaktbringens einer Enzymlösung, die Allulose-Epimerase mit einer spezifischen Aktivität von 50 U/mg oder mehr enthält, mit einem schwach basischen Anionenaustauscherharz auf Styrolbasis vom porösen Typ oder einem schwach basischen Anionenaustauscherharz auf Styrolbasis vom Geltyp, so dass die gesamte beladene Proteinmenge 2 bis 15 mg/ml-R beträgt,
wobei eine lonenaustauschgruppe des schwach basischen Anionenaustauscherharzes auf Styrolbasis vom porösen Typ oder des schwach basischen Anionenaustauscherharzes auf Styrolbasis vom Geltyp ein tertiäres Amin ist,
wobei die Anzahl der Allulose-Epimerase-Einheiten der Enzymlösung, die mit dem lonenaustauscherharz in Kontakt gebracht werden soll, 270 U oder mehr pro 1 ml des Ionenaustauscherharzes beträgt,
wobei die spezifische Aktivität der immobilisierten Allulose-Epimerase 200 bis 500 U/ml-R beträgt, und
wobei die spezifische Aktivität der Allulose-Epimerase gemäß dem in der Beschreibung offenbarten Verfahren gemessen wird.

2. Verfahren nach Anspruch 1, wobei eine lonenaustauschgruppe des schwach basischen Anionenaustauscherharzes auf Styrolbasis vom porösen Typ -N(CH₃)₂ ist.

## Revendications

1. Procédé de production d'une allulose épimérase immobilisée comprenant une étape consistant à amener une solution enzymatique contenant de l'allulose épimérase ayant une activité spécifique de 50 U/mg ou plus en contact avec une résine échangeuse d'anions faiblement basique de type poreuse à base de styrène ou une résine échangeuse d'anions faiblement basique de type gel à base de styrène de sorte que la quantité protéique chargée totale est de 2 à 15 mg/ml-R,
dans lequel un groupe échangeur d'ions de la résine échangeuse d'anions faiblement basique de type poreuse à base de styrène ou la résine échangeuse d'anions faiblement basique de type gel à base de styrène est une amine tertiaire,
dans lequel le nombre d'unités d'allulose épimérase de la solution enzymatique devant être amenée en contact avec la résine échangeuse d'ions est de 270 U ou plus pour 1 ml de la résine échangeuse d'ions,
dans lequel l'activité spécifique de l'allulose épimérase immobilisée est de 200 à 500 U/ml-R, et
dans lequel l'activité spécifique de l'allulose épimérase est mesurée conformément au procédé divulgué dans la description.

2. Procédé selon la revendication 1, dans lequel un groupe échangeur d'ions de la résine échangeuse d'anions faiblement basique de type poreuse à base de styrène est -N(CH₃)₂.
